Europäisches Patentamt

European Patent Office

Office européen des brevets

(19)

(11) **EP 1 618 887 A1**

(12) **EUROPEAN PATENT APPLICATION**

(43) Date of publication:
**25.01.2006 Bulletin 2006/04**

(51) Int Cl.:
*A61K 38/16* (2006.01)

(21) Application number: **04077013.3**

(22) Date of filing: **12.07.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(71) Applicant: **UMC Utrecht Holding B.V.**
**3584 CK Utrecht (NL)**

(72) Inventor: **van Dijk, Hans**
**3732 HC De Bilt (NL)**

(74) Representative: **Winckels, Johannes Hubertus F. et al**
**Vereenigde**
**Johan de Wittlaan 7**
**2517 JR Den Haag (NL)**

(54) **Clearance of polyols from the body**

(57)    The invention discloses the use of MBL or a functional fragment and/or derivative and/or functional equivalent thereof in the preparation of a medicament for enhancing the removal of a substance having at least one available hydroxyl group or a complex comprising such a substance from a body fluid such as for example blood, lymph or serum in a subject or outside of the body. The invention further discloses said methods and use for the clearance of a polyol, a nucleic acid, a nucleosome, a complement activating part or antibody, and/or a C3 and/or C4 activator. The invention also provides the use of said compounds for the prevention and /or amelioration of autoimmune and infectious diseases and of SLE. The invention also provides substances binding to MBL and comprising S-glycopeptides and N-glycopeptides and methods to produce said S-glycopeptides and N-glycopeptides.

EP 1 618 887 A1

**Description**

The invention relates to the clearance of certain molecules comprising free (meaning available) hydroxyl groups from body fluids. More in particular, the invention relates to the removal of substances or complexes of substances having several free hydroxyl groups (referred to in here in general as polyols) from body fluids, particularly from the circulation. The present invention provides the teaching that organisms have a transport and clearance system for substances that are not supposed to be in the circulation. A common denominator of this system is that it is capable to bind substances that have free hydroxyl groups, such as carbohydrates, polysaccharides (such as those present on micro-organisms), nucleic acids, lipoproteins and the like. Each of these hydroxyl containing substances, or complexes of substances has been linked to pathological conditions. In the case of polysaccharides there is a link to e.g. infectious diseases; for nucleic acids there is a link with e.g. autoimmune diseases and for carbohydrates there is at least one link with cardiovascular diseases. Since the transport and/or the clearance of these substances uses for at least a part the same system, this invention also teaches to increase efficacy of removal of certain hydroxyl group containing substances (or complexes) by diminishing the presence in the same body fluid of other groups containing free hydroxyl groups.

In recent literature, the binding and transport of lipoproteins and carbohydrates in the body has been ascribed to the presence and action of Mannose Binding Lectin (MBL). MBL is a high-molecular-weight protein, present in blood plasma in minute amounts (normal value about 1.7 microgram per ml). MBL belongs to the family of collagenous lectins (collectins), together with human CL-L1 (2), CL-P1 (3), lung surfactant proteins A and D and the bovine proteins conglutinin (4), collectin-43 (5) and collectin-46 (6). Upon binding to foreign bodies or lipoprotein, MBL undergoes a conformational change leading to the coordinate activation of at least two MBL-associated proteins (MASP-1, and MASP-2), which starts the lectin pathway (LP) of complement activation. MBL mediates the recognition of foreign particles by its three identical sugar-binding domains with specificity for mannose, N-acetylglucosamine, and fucose (7). A cellular receptor for MBL has only recently been recognized, i.e.CR1 (= complement receptor 1 = CD35 = C3b/C4b receptor) that is present on the surface of erythrocytes and inflammatory cells (8). This receptor also binds C3b and C4b molecules and the complex formed by C3b and C4b. The binding of MBL, or of C3b or C4b or the complex C3b-C4b to said molecule or organism facilitates the binding to CR1 on erythrocytes. Said binding to erythrocytes facilitates the transport of said organism or molecule through the circulation to the spleen or the liver where further degradation or conversion of said organism or molecule occurs. This pathway has been described in patent applications WO02/22160 and WO02/22161.

MBL deficiencies are quite common in man. In fact, low-level haplotypes can occur in up to 30% of the human population (14) and are associated with all kinds of infectious, atherosclerotic and infection-related diseases. MBL treatment of individuals with recurrent infections secondary to severe MBL deficiency has proven to be useful in certain cases (15). Determining MBL deficiency is not an easy task. Over the past twelve years, several enzyme immunoassays have been developed to estimate antigenic MBL levels in serum. One common disadvantage of these antigenic assays, however, is that they fail to measure functional MBL, i.e. the joint activity of the MBL-MASP's complex. Recently a haemolytic test for the estimation of functional MBL in serum has been reported (16). The normal value of MBL in serum ranges from 0.42 microgram MBL equivalents per ml to 1.67 microgram MBL equivalents per ml, low serum MBL ranges from 0.2 microgram MBL equivalents per ml to 0.42 microgram MBL equivalents per ml, and below 0.2 microgram MBL equivalents per ml is considered completely MBL deficient. The incidence of functional MBL deficiency is 5 % of the population. (16).

MBL recognises mannose, fucose and/or N-acetylglucosamine by its specific sugar binding groups. Until now, the nature of these binding groups has not been fully clear. Herein the common denominator for the binding specificity for mannose, fucose and N-acetylglucosamine is determined as the presence of an available hydroxyl group. All sugars contain a hydroxyl group in addition to either a ketone or an aldehyde group. The hydroxyl group of one sugar may combine with the ketone group or the aldehyde group of another sugar molecule to form a disaccharide. This process is not only possible for hexose sugars, which contain 6 C-atoms, but also for pentose sugars (containing 5 C-atoms) and for sugars with another number of C-atoms. Thus, the invention provides the use of the MBL system and/or elements thereof for the removal, or clearance of substance having a free hydroxyl group from a body fluid.

An example of a substance comprising available hydroxyl groups is pentose. Pentose is a building block of DNA. DNA is present in cell nuclei and when cellular material is transported after apoptosis or cell death, DNA fragments are recognised by the MBL system and are bound by C3B and C4B for binding to the CR1 receptor on erythrocytes. In this way, the DNA fragments are transported to the liver and the spleen for elimination and recycling. The removal of DNA fragments may be decreased in individuals with (partial) MBL deficiency, or in individuals having a malfunctioning of the MBL activation cascade because another factor in the cascade is missing or malfunctioning, like for example, in an individual with C4A deficiency. Other reasons for the presence of DNA in circulation may also exist.

Although SLE has been related to deficiencies in the early components of the complement system such as MBL, C1, C2 and C4 (1-8, p88), the mechanism behind this correlation has never been elucidated. The present invention discloses that a DNA fragment, possibly still complexed with a histone protein, with which it was in close contact during the incorporation in the DNA, can be present as a DNA fragment or nucleosome in a body fluid. Said body fluid may comprise blood, and/or serum, and/or tissue fluid, and/or lymph fluid. Prolonged presence of a DNA fragment, and/or a nucleosome in the circulation may trigger the body to an immunological reaction. The immune system starts to produce antibodies, or even activated T cells that react to said DNA fragment and/or nucleosome. This process results in the formation of a DNA fragment and/or nucleosome complexed with antibodies. These complexes activate complement, but because of a malfunctioning or overload of the MBL system, the complexes are not transported to the liver or spleen as fast as they would in a normal individual. In this way, autoimmune disease may develop. Therefore, the activated complement provokes bystander effects like for example lysis of erythrocytes, or infiltration of vessel walls by inflammatory cells. Examples of an autoimmune disease with complex formation between DNA fragments and antibodies are Systemic Lupus Erythematosus (SLE), autoimmune Hemolytic Anemia, or Autoimmune Thyreoiditis. Increasing the level of functional MBL or another complement factor that is necessary for the transportation of the DNA fragments and the complexes by the erythrocytes to the spleen and liver can break this chain of events. Any nucleic acid that is present in a body fluid, particularly in circulation, can be made to clear at an enhanced rate by providing at least one element (particularly a rate- limiting element) from the MBL pathway. This is true in body fluids of MBL-deficient, low MBL as well as normal MBL individuals.

Herein we disclose the use of MBL or a functional fragment and/or derivative and/or functional equivalent thereof in the preparation of a medicament for enhancing the removal of a substance having at least one available hydroxyl group or a complex comprising such a substance from a body fluid.

Of course, this action is also achieved by increasing the level of a functional fragment and/or derivative and/or functional equivalent of MBL or another complement factor. A functional fragment of MBL means a part of the MBL molecule which still exerts at least part of the function of the whole MBL molecule, a derivative of MBL being defined as a protein which has been altered such that the biological properties of said molecule are essentially the same in kind, not necessarily in amount. A derivative can be provided in many ways, for instance through conservative amino-acid substitution. An equivalent of MBL is a molecule having the same biological function in kind, not necessarily in amount, like for example the C3b-C4b complex, and/or C3b and/or C4b. Substances or molecules having the same biological function can be selected by using the method of MBL activation as described in the examples. Of course, antibodies or functional part or derivatives thereof, that bind to a substance having a hydroxyl group may also activate the complement cascade and help in clearing said substance and/or a DNA fragment(s) by binding it to erythrocytes. A functional part of an antibody is defined as a part, which has the same biological properties in kind, not necessarily in amount. By biological properties is meant the capability to bind to an antigen. A functional derivative of an antibody is defined as a protein, which has been altered such that the biological properties of said molecule are essentially the same in kind, not necessarily in amount. A derivative can be provided in many ways, for instance through conservative amino-acid substitution. Such an MBL or a functional fragment and/or derivative and/or functional equivalent thereof will be used to produce a medicament for enhancing the removal of a substance having at least one available hydroxyl group or a complex comprising such a substance from a body fluid. Said body fluid may comprise blood, lymph fluid or serum. We teach the use of MBL or a functional fragment and/or derivative and/or functional equivalent thereof in the preparation of a medicament for enhancing the removal of a substance having at least one available hydroxyl group or a complex comprising such a substance from a body fluid, wherein said body fluid is blood, lymph fluid or serum. Of course said body fluid may be part of a human being, therefore, we disclose the use of MBL or a functional fragment and/or derivative and/or functional equivalent thereof in the preparation of a medicament for enhancing the removal of a substance having at least one available hydroxyl group or a complex comprising such a substance from a body fluid in a subject.

Care has to be taken that the MBL pathway is indeed available for removing or enhancing the removal of the substance or complex that needs to be removed. It is important that no other candidates for removal by the MBL system are present in any rate-limiting amount for the substance and/or complex that needs to be removed. Since the common denominator for binding has been identified herein, this determination is now within the reach of the person skilled in the art. It is also taught to use MBL or a functional fragment and/or derivative and/or functional equivalent thereof in the preparation of a medicament for enhancing the removal of a nucleic acid, and/or a nucleosome having at least one available hydroxyl group or a complex comprising such a nucleic acid and/or nucleosome from a body fluid.

Enhancing transportation of a nucleic acid, particularly a DNA molecule or fragment thereof and/or a complex comprising a DNA (RNA) molecule or fragment can be achieved if such a molecule or complex has a free hydroxyl group. The presence of nucleic acids in circulation is associated with pathological conditions such as infection, inflammation and/or autoimmune disease, therefore, the present invention discloses the use of MBL as described above, especially for the prevention and/or amelioration of autoimmune diseases. Because SLE is an autoimmune disease characterized by the presence of complexes in the circulation of DNA fragment with antibodies, an individual with SLE would benefit from the treatment as mentioned above. Therefore, the invention discloses the use of MBL as described above for the prevention and/or amelioration of SLE. A polyol may also be a constituent of a micro-organism with pathogenic potential, like for example a mannose or an N-acetylglucosamine moiety or another carbohydrate molecule with at least one available hydroxyl group (e.g. fucose). Therefore, the invention also teaches the use as described above for the treatment of infectious diseases.

The invention provides a common denominator of substances, complexes, micro-organisms and/or particles that can be transported and/or cleared from the circulation by the MBL pathway. This common denominator is the presence of at least one, or preferably more free hydroxyl groups. Not every substance in circulation is a candidate for removal because its presence is wanted or has free hydroxyl groups or insufficient free hydroxyl groups to be able to be transported by the MBL pathway. The invention therefore provides methods and means to provide at least certain substances with free hydroxyl groups. In general many substances that are provided to the circulation can be provided with free hydroxyl groups at sites that do not affect their function, so that their clearance can be enhanced. Particularly, drugs containing free hydroxyl groups can be cleared sooner by boosting the MBL pathway as described herein above. It is also possible to provide molecules or complexes with free hydroxyl groups in situ. One can provide to the circulation of a subject a molecule comprising free hydroxyl groups and having specific affinity for a molecule or complex to be transported and/or cleared by the MBL pathway. Upon binding, the complex can then be removed by the MBL pathway as present or by a boosted MBL pathway.

An example of such a molecule with specific affinity for a complex in the circulation is a fatty acid provided with a sugar moiety (possibly through a linker). When such a molecule is provided in the circulation preferably by incorporation at the level of the mucosa of the gut wall in a lipoprotein, more preferably along with a phospholipid, it will be taken up by fatty particles, such as chylomicrons and provide these with (additional) moieties recognised by the MBL system. These particles are therefore better removed from the circulation, particularly if the MBL pathway is upregulated.

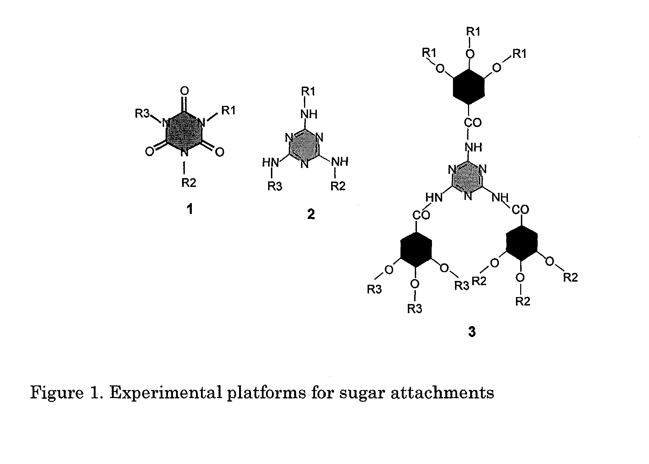

Thus the invention provides a substance comprising a saccharide core, at least one hydrophobic tail and at least one free hydroxyl group, for use in providing a fatty acid complex in a subject with additional free hydroxyl groups. In the alternative, the invention provides a substance comprising a scaffold as presented in fig.1, provided with at least one hydroxyl group and at least one apolar region for use in providing a fatty acid complex in a subject with additional free hydroxyl groups. These substances themselves are also part of the present invention. Their use is clear. The fatty acid tail and/or apolar region is able to incorporate into a fatty acid layer or membrane-like structure. The sugar moiety and/or free hydroxyl group will stick out and can be bound and processed by the MBL pathway. Many sugar moieties are possible. A high throughput screen testing against the ability to bind MBL is used to identify the best sugar moieties and/or free hydroxyl group presenting moieties. The best fatty acid tail is synthesised and selected for best ability to pass from the gastrointestinal tract to the circulation and incorporation in the fatty acid particles intended.

Preferably, the bond (albeit indirect) between apolar region and free hydroxyl group(s) is resistant to breakdown in the stomach so that the substance can be taken orally. As said before, the MBL system must be available for clearing and/or transporting the desired substance, so inhibitors of the MBL pathway should be present in the lowest amounts possible. One such a known inhibitor is glucose. Thus the invention provides a use according to the invention, wherein the glucose levels in said subject or body fluid are reduced.

In a more preferred embodiment, a glycopeptide, which contains a monosaccharide, mimics a complex glycan ligand. The combinatorial synthesis, via the split-and-mix approach, and solid-phase screening of O-glycopeptide libraries has been established as an effective tool for the identification of a high-affinity ligand for a dedicated receptor and protein. In another embodiment of the invention, a combinatorial library of S-glycopeptide (glycopeptide in which the connecting oxygen between the carbohydrate and peptide backbone is replaced by a sulphur) is disclosed. The compounds present in a library of S-glycopeptide have several advantages over naturally occurring complex glycans:

    1. Increased *in vivo* stability, esp. in the stomach
    2. Higher affinity for receptors than natural ligands and,
    3. The S-glycopeptides can be generated via solid-phase combinatorial library synthesis.

Instead of a replacement of the oxygen atom by a sulphur atom, a nitrogen atom can also replace the oxygen. Therefore, in a preferred embodiment, the invention discloses a combinatorial N-glycopeptide library.

We developed S- and N-glycopeptides for in vivo drug stability, rather than the unstable O-glycopeptides. Synthesis of a mannose-containing S-glycopeptide or N-glycopeptide library generates thousands of possible high-affinity ligands for the healthy and truncated mannose-binding lectin (MBL). Therefore, the invention also discloses a S-glycopeptide and/or an N-glycopeptide from a library of the invention. Solid-phase high-throughput-screening of this library with fluorescent-labelled MBL will identify specific, in vivo stable ligands for MBL.

As said before, the invention can be practiced in MBL- normal, MBL-low and MBL-deficient subjects. In all three it is advantageous to boost the MBL pathway for removal of substances of complexes. For the removal of fatty acid complexes it is thus preferred to provide a boost to the MBL pathway, preferably MBL or a derivative, fragment, etc. thereof and to also provide enhanced free hydroxyl groups on the fatty acid complexes to be removed. Thus the invention provides a kit of parts comprising a preparation comprising MBL or a functional fragment and/or derivative and/or functional equivalent thereof and a preparation comprising a substance comprising a saccharide core, at least one hydrophobic tail and at least one free hydroxyl group, as well as a kit of parts comprising a preparation comprising MBL or a functional fragment and/or derivative and/or functional equivalent thereof and a preparation comprising a substance comprising a scaffold as presented in fig.1, provided with at least one hydroxyl group and at least one apolar region.

The invention will be illustrated in the following experimental part.

### Example 1

### High-throughput synthesis of glycosylated peptides

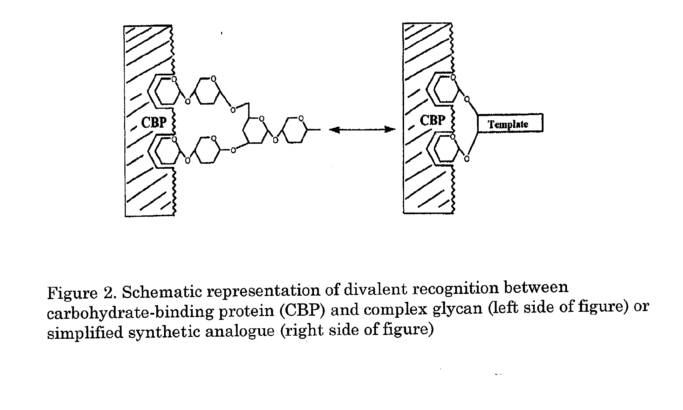

It has been shown that not all carbohydrate residues of a complex glycan contribute equally to the interaction with a lectin. Essentially, only those epitopes of glycans that are exposed to the external environment are involved in interactions. Therefore, simplified synthetic molecules can be used to mimic complex glycan ligands in protein carbohydrate interaction studies (see fig. 1 and 2). It has unambiguously been shown that simple, monosaccharide-containing glycopeptides are good mimics of complex glycan ligands. The combinatorial synthesis, via the split-and-mix approach, and solid-phase screening of O-glycopeptide libraries has been established as an effective tool for the identification of high-affinity ligands for dedicated receptors and proteins.

One possible problem of this approach is the presence of the oxygen, linking the carbohydrate to the peptide backbone, towards both acidic conditions and hydrolytic enzymes present in the human circulation (esp. the GE-tract). In traditional carbohydrate synthesis it has been shown that replacing the oxygen linkage between two monosaccharides by a sulphur linkage renders the oligosaccharide stabile towards both enzymatic hydrolysis and acidic conditions.

The present invention discloses the combinatorial synthesis of S-glycopeptide (glycopeptides in which the connecting oxygen between the carbohydrate and peptide backbone is replaced by a sulphur) libraries. The compounds present in this type of library have several advantages over naturally occurring complex glycans:

1. Increased *in vivo* stability, esp. in the stomach
2. Higher affinity for receptors than natural ligands and,
3. The S-glycopeptides can be generated via solid-phase combinatorial library synthesis.

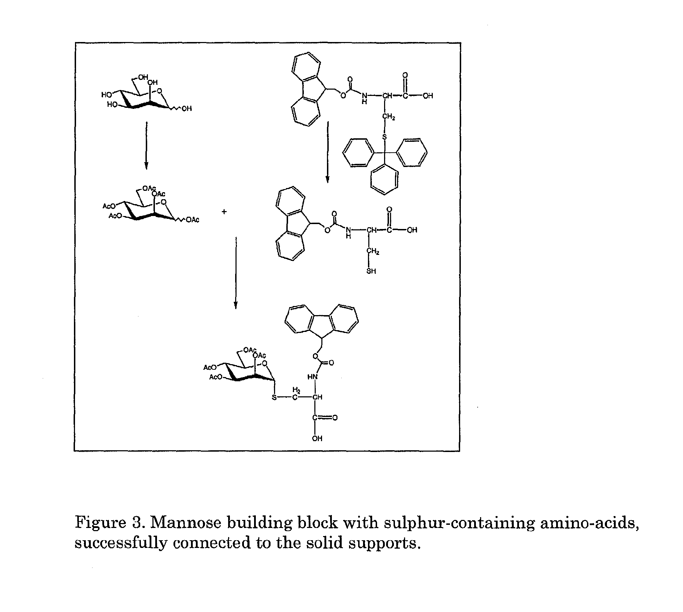

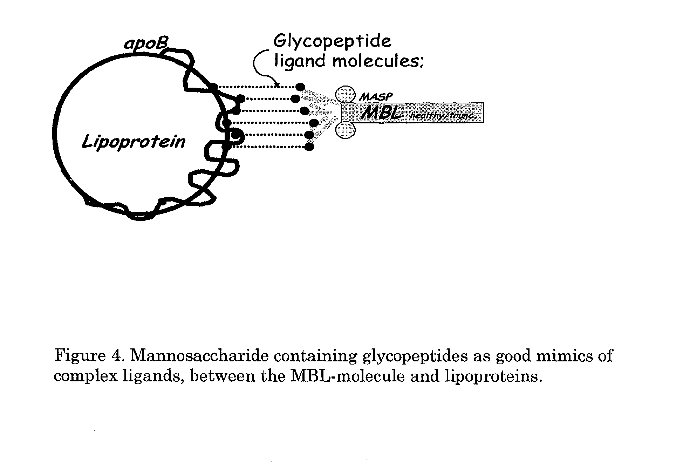

We developed S- and N-glycopeptides for in vivo drug stability, rather than the unstable O-glycopeptides. Synthesis of a mannose-containing S-glycopeptide library generates thousands of possible high-affinity ligands for the healthy and truncated mannose-binding lectin (MBL), see fig. 3. Solid-phase high-throughput-screening of this library with fluorescent-labelled MBL will identify specific, in vivo stable ligands for MBL (fig. 4).

The most effective and applicable ligands are coupled to the most favourite fatty backbone, as described hereunder. Conjugated to a fatty backbone, the obtained complex increases the number of naturally occurring glycoproteins at the surface of the lipoproteins in the most natural way. The complex should be embedded into the circulating lipid-particles *via* the fatty backbone, while the S-glycopeptide part (hydrophilic) will be on the outside of the lipid-particle. This is most logical as the inside of the lipoproteins is highly lipophilic. MBL can bind, *via* the S-glycopeptide, with high affinity to the lipid-particle and help to clear it from the circulation.

### Example 2

### High-throughput synthesis of lipids/carbohydrates

We prepared compounds that have a lipophilic backbone to which different sugar moieties can attach for incorporation into lipoproteins to increase the linking-to MBL. The molecular platform is lipophilic to facilitate the easy incorporation into the single membrane of the lipid particle.

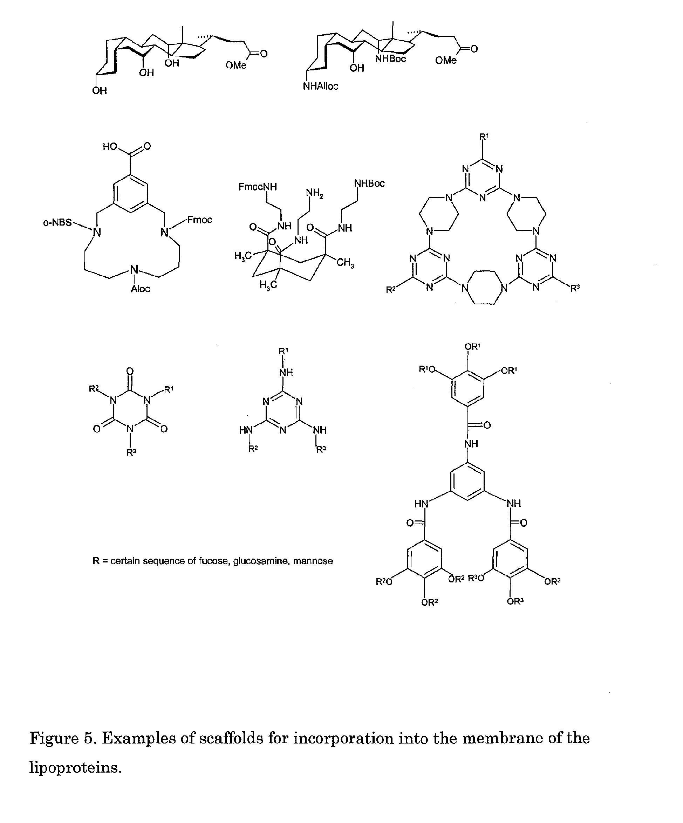

A selection of the scaffolds used is summarized in Figure 5. At first instance known scaffolds have been chosen, viz. steroid derivatives (Davis c.s., Tetrahedron Lett. 1999, 40, 2849), triazacyclophane derivative (Opatz and Liskamp, J. Comb. Chem. 2002, 4, 275), Kemp's triacid derivative (Kocis c.s., Tetrahedron Lett. 1995, 36, 6623), and a triazine-based macrocyclic scaffold (Löwik and Lowe, Tetrahedron Lett. 2000, *41*, 1837). In addition, cyanuric acid and melamine are used as a platform to assemble sugars. Further development will result in small synthetic wedges decorated with sugars. Also a series of long-chain saturated and (multiple) unsaturated fatty acids is used as scaffolds, with and without spacers between the lipid structure and the carbohydrate groups. Spacers have benefits to prevent splitting off the carbohydrate groups by endogenous carbohydrates. Series of such compounds are synthesized to determine the optimum compounds with respect to non-immunogenicity.

Such scaffolds are used to investigate the level of incorporation of the compounds with conservation of the sugar moieties on the scaffolds and to establish the correct presentation at the outer surface of the lipoproteins. Scaffolds with one and with more sugars groups are compared, to explore the parameters necessary for the carbohydrate groups to be externalised.

At first instance different libraries are prepared by covalent assembly of sugars in a well-defined 3D-orientation on a scaffold. A more controlled variation of the 'sugar sequence' is applied based on the hits found in the screening test in order to end up with one or more compounds with an optimum activity.

*Example 3*

*High-throughput in vitro assay method number 1*

We developed (and published) a haemolytic assay for the estimation of functional mannose-binding lectin levels in human serum.

The functional MBL assay, based on the principle of MBL-dependent bystander haemolysis, was executed in U-well microtitre plates (Greiner, Frickenhausen, Germany). Firstly, sera to be tested were serially diluted in the test plate in order to obtain 50 microlitre samples in a dilution series of $10^{-0,5}$ (1/10 - 1/ 3162), starting with a serum dilution of 10% in VBS$^{++}$. Then, a 10 ml test mixture in VBS$^{++}$ was prepared per microtitre plate. Such a mixture contained a standardized amount of freshly cultured baker's yeast S. cerevisiae (3.0 x$10^7$ cells), 150 microlitre reagent serum as a source of all complement components except MBL, and $10^9$ ChE as the target cells of bystander haemolysis. Direct haemolysis of the erythrocytes induced by yeast cells was excluded by incubating the erythrocytes and yeast cells together just with reagent serum. Human pooled serum (MBL titre 1.67 microgram/ml as determined by ELISA) (Bax et al., 1999) was used as the MBL reference sample in the assay. A total of 100 microlitre of test mixture was added to each test well containing serum dilutions, and the microtitre plate was put on a water-based incubator operating at 37 °C (Klerx et al., 1983). After incubation, the erythrocytes were spun down and 50 microlitre samples of each supernatant were transferred to a flat-bottom plate, with 200 microlitre samples of water in each well. Haemoglobin release was measured in an ELISA reader operating at a wavelength of 405 nm. Percentages of haemolysis were calculated using controls for 100% (water lysed) and 0% (buffer control) haemolysis. The percentages were then transformed to the number of active sites per cell according to the equation published by Borsos and Rapp (1963):

$$Z \text{ (number of active sites per cell)} = \ln(1 - \text{fraction erythrocytes lysed})$$

Titres were read at Z= 0.200. Functional MBL titres were defined as titres in microgram equivalent MBL per ml relative to the known reference. Sera were also tested in EGTA-VB to study alternative pathway activation and in EDTA that inhibits the three complement activation pathways. For this and all other results presented, a minimum of three replicate assays was performed. The inter-assay and intra-assay variations (n ≥ 10) were determined as well.

Inhibition of MBL activation in the functional assay by competing carbohydrates

This was done by pre-incubating test serum samples with mannose, GlcNAc, or galactose at 37 °C for 30 min, starting with a concentration of 2.5 mg carbohydrate per well. A checkerboard titration was done in the following manner: HPS was diluted in all the horizontal rows of the microtitre plate, and dilutions (1/3) of the carbohydrates were tested (833, 278, 92.6, 30.9, 10.3, and 3.4 microgram/well) in the vertical rows.

This "stand-alone" assay is restructured into a high-throughput mode, in order to effectively screen large series of new compounds from the chemical synthesis programs. This test system is based on the function of MBL as an activator of complement resulting in haemolysis of erythrocytes ( *Kuipers S, Aerts PC, Sjoholm AG, Harmsen T, van Dijk H. A haemolytic assay for the estimation of functional mannose-binding lectin levels in human serum. J Immunol Methods 2002; 268:* 149-157).This assay was developed to estimate functional mannose-binding lectin (MBL) levels in serum based on the principle of yeast-induced bystander lysis of chicken erythrocytes (ChE). The assay is well suited for the large-scale testing of patient samples for a functional MBL pathway of complement activation and for testing of activity of (new) compounds in the CLiP-pathway.

*Example 4*

*High-throughput in vitro assay method number 2*

We have established a second in vitro assay, based on a different mechanism. This in vitro (stand alone) assay has been developed recently and makes use of the novel discovered binding capacity of apoB-containing particles to cells provided with the human CR1-receptor, similar to the surface of the human red blood cells. In brief, an immortalized cell line (CHO-cells) was transfected with the hCR1 gene. Incubations with opsonized apoB-containing particles were performed on hCR1 expressing cells and control (non-hCR1) CHO-cells. The data clearly show an increase of the apoB signal on hCR1-transfected CHO cells, measured by FACS (mean ± SD of 2 independent experiments). These data illustrate that apoB-containing particles bind to hCR1. Binding of these particles to hCR1 in this cell line can be used as parameter to monitor CLiP inducing activity by lead compounds.

Caco-2 cells, colon carcinoma cells are well-suited to determine the incorporation of lipid/carbohydrate compounds into lipoproteins in vitro. Such cells are well-suited both for the in vitro qualitative analysis of the incorporation, as well as the quantitative yield of the incorporation (flux), for which fluorescence labelled compound will be prepared. Caco-2-cells are immortalized, colon carcinoma cells that are used as a model to study chylomicron synthesis in *vitro.* This cell culture model is polarized similarly to human intestinal cells with an apical (uptake of nutrients) and a basolateral (secretion of intracellularly synthesized products, i.e. chylomicrons) membrane. In order to investigate whether the compounds that will be developed are indeed incorporated into chylomicrons, we will use this model. Incubations with these compounds on confluent cell lines will be carried out and lipoproteins synthesized by these cells will be recovered in the medium. After isolation of these lipoproteins, direct quantification will be carried out i.a. with HPLC. In addition, it will be possible to use these lipoproteins in our MBL activation assay in order to evaluate which of these incorporated compounds will induce the best MBL activation in vitro.

### Example 5

### *In vivo assay, transgenic mouse programme*

In order to investigate the exact mechanisms by which apoB-containing lipoproteins bind to hCR1 on the erythrocytes, hCR1 transgenic mice are developed. Transfections of several hCR1 constructs have been performed into cell lines that have no endogenous hCR1 expression including COS and CHO cells). Based thereon, a transgenic mouse model with human CR1 expressed by the murine erythrocytes is obtained and subsequently cross bred with an APO E knockout mouse. This genetic model facilitates the analysis of the protective effect of erythrocyte CR1 on atherogenesis. Furthermore, the effect of several (pharmacological) interventions on the development of atherosclerosis can be tested in this animal model.

### References:

Crego Calama, M.; Hulst, R.; Fokkens, R.; Nibbering, N.M.M.; Timmerman, P.; Reinhoudt, D.N., Libraries of non-covalent hydrogen-bonded assemblies; combinatorial synthesis of supramolecular systems, Chem. Commun. 1998, 1021-1022.

Timmerman, P.; Reinhoudt, D.N., A combinatorial approach to synthetic receptors, Adv. Mater. 1999, 11, 71-74.

Crego Calama, M.; Timmerman, P.; Reinhoudt, D.N., Guest-templated selection and amplification of a receptor by noncovalent combinatorial synthesis, Angew. Chem. Int. Ed. 2000, 39, 755-758.

De Jong, M.R.; Knegtel, R.M.A.; Grootenhuis, P.D.J.; Huskens, J.; Reinhoudt, D.N., A method to identify and screen libraries of guests that complex to a synthetic host, Angew. Chem. Int. Ed. 2002, 41, 1004-1008.

K.M. Halkes, P.M.St. Hilaire, A.M. Jansson, C.H. Gotfredsen and M. Meldal, Synthesis and application of sialic acid-containing building blocks for glycopeptide libraries. Establishing glycosylation conditions. J. Chem. Soc., PT1, 2000, 2127-2133

K.M. Halkes, P.M.St. Hilaire, P.R. Crocker and M. Meldal, Glycopeptides as oligosaccharide mimics: high affinity sialopeptide ligands for sialoadhesin from combinatorial libraries. J. Comb. Chem. 5, 2003, 18-27

Kuipers S, Aerts PC, Sjoholm AG, Harmsen T, van Dijk H. A haemolytic assay for the estimation of functional mannose-binding lectin levels in human serum. J Immunol Methods 2002; 268: 149-157.

Persson L, Borén J, Robertson A-K, Wallenius V, Hansson GK, Pekna M. Lack of Complement Factor C3, but Not Factor B, Increases Hyperlipidemia and Atherosclerosis in Apolipoprotein E-/- Low-Density Lipoprotein Receptor-/Mice. Arterioscler Thromb Vasc Biol 2004; 24: 1-7

Buono et al. Influence of C3 deficiency on atherosclerosis. Circulation 2002; 105: 3025-3031

Castro Cabezas, de Bruin TWA, de Valk HW, Shoulders CC, Jansen H, Erkelens DW. Impaired fatty acid metabolism in familial combined hyperlipidemia. A mechanism associating hepatic apolipoprotein B overproduction and insulin resistance. J Clin Invest 1993; 92: 160-8.

Erkelens DW, de Bruin TWA, Castro Cabezas M. Tulp Syndrome. The Lancet 1993; 342: 1536-1537.

Castro Cabezas M, Erkelens DW. The direct way from gut to vessel wall: Atheroinitiation. Eur J Clin Invest 1998; 28: 504-505.

Castro Cabezas M, Erkelens DW. Triglycerides and atherosclerosis: To feast or fast. Neth J Med 2000; 56: 110-118.

De Man FHAF, Castro Cabezas M, van Barlingen HHJJ, Erkelens DW, de Bruin TWA. Metabolism of triglyceride-rich lipoproteins in noninsulin-dependent diabetes mellitus: Relation to atherosclerosis. Eur J Clin Invest 1996; 26: 89-108.

Van Wijk JPH, Castro Cabezas M, Halkes CJM, Erkelens DW. Effects of different nutrient intakes on daytime triacylglycolemia in healthy, normolipemic, free-living men. Am J Clin Nutr 2001; 74: 171-8.

Castro Cabezas M, Halkes CJM, Erkelens DW. Obesity and postprandial free fatty acids: Double trouble. Nutr Metabol Cardiovasc Risk Factors 2001; 11: 134-142.

Meijssen S, van Dijk H, Verseyden C, Erkelens DW, Castro Cabezas M. Delayed and exaggerated postprandial complement 3 response in familial combined hyperlipidemia. Arterioscl Thromb Vasc Biol 2002; 22: 811-816.

Halkes CJM, van Dijk H, De Jaegere PPTh, Plokker HWM, Erkelens DW, Castro Cabezas M. Postprandial increase of complement component 3 in normolipidemic patients with coronary artery disease. Effects of expanded dose simvastatin. Arterioscl Thromb Vasc Biol 2001; 21: 1526-1530.

Van Oostrom AJHHM, Van Dijk H, Verseyden C, Sniderman AD, Cianflone K, Rabelink TJ, Castro Cabezas M. A mixed meal prevents the fat-mediated postprandial rise of complement component 3 by enhanced insulin-mediated free fatty acid trapping. Am J Clin Nutr (2003, in press).

Halkes CJM, Van Dijk H, De Jaegere PPTh, Plokker HWM, Erkelens DW, Castro Cabezas M. Gender differences in postprandial ketone bodies in normolipidemic subjects and in untreated patients with familial combined hyperlipidemia. Arterioscl Thromb Vasc Med 2003 (in press).

Verseyden C, Meijssen S, van Dijk H, Jansen H, Castro Cabezas M. Effects of atorvastatin on fasting and postprandial complement component 3 response in familial combined hyperlipidemia. J Lipid Res 2003 (in press).

Madsen, HO, Videm, V, Svejgaard, A, Svennevig, JL, Garred, P. Association of mannose-binding lectin deficiency with severe atherosclerosis. Lancet 1998; 352:959-960.

Reitsma JB, Bonsel G.J. Hart- en vaatziekten in Nederland 2001. Cijfers over ziekte en sterfte, de Nederlandse Hartstichting. Den Haag: 2001.

Rosamond WD, Chambless LE, Folsorn AR, Cooper LS, Conwill DE, Clegg L, Wang CH, Heiss G. Trends in the incidence of myocardial infarction and in mortality due to coronary heart disease, 1987 to 1994. N Eng J Med 1998.339:861-867.

Nestel P. Relationship between plasma triglycerides and removal of chylomicrons. J Clin Invest 1964;43:943-949.

Zilversmit DB. Atherogenesis: a postprandial phenomenon. Circulation 1979;60:473-485.

Austin MA, Hokanson JE, Edwards KL. Hypertriglyceridemia as a cardiovascular risk factor. Am J Cardiol 1998;81:7B-12B.

Stavenow L, Kjellstrom T. Influence of serum triglyceride levels on the risk for myocardial infarction in 12,510 middle aged males: interaction with serum cholesterol. Atherosclerosis, 1999;147:243247.

Hulse M, Gershberg H. Variability in blood cholesterol, triglycerides, tree fatty acids, glucose and body weight in maturity-onset diabetics. Am J Med Sci 1969;258:114-120.

Karpe F, Hamsten A. Postprandial lipoprotein metabolism and atherosclerosis. Curr Opin Lipidol 1995;6:123-129.

Ross R. Atherosclerosis - an inflammatory disease. N Engl J Med 1999;340:115-126.

Skalen K, Gustafsson M, Knutsen Rydberg E, Mattson Hulten L, Innerarity TL, Borén J. Subendothelial retention of atherogenic liprotein particles in early atherosclerosis. Nature 2002; 417: 750-754.

Schonfeld G. The hypobetalipoproteinemias. Annu Rev Nutr 1995; 15: 23-34.

Lusis AJ. Atherosclerosis. Nature 2000;407:233-241.

Meilinger M, Gschwentner C, Burger I, Haumer M, Wahrmann M, Szollar L, Nimpf J, Huettinger M. Metabolism of activated complement component C3 is mediated by the low density lipoprotein receptor-related protein/$\alpha$2-macroglobulin receptor. J Biol Chem 1999; 274: 38091-38096.

Cianflone KM, Sniderman AD, Walsh MJ, Vu HT, Gagnon J, Rodriguez MA. Purification and characterization of acylation stimulating protein. J Biol Chem 1989;264:426-30.

Cianflone K, Maslowska M, Sniderman AD. Acylation stimulating protein (ASP), an adipocyte autocrine: new directions. Semin Cell Dev Biol 1999;10:31-41.

Cianflone KM, Sniderman AD, Walsh MJ, Vu HT, Gagnon J, Rodriguez MA. Purification and characterization of acylation stimulating protein. J Biol Chem 1989; 264: 426-430.

Walport MJ. Complement. N Engl J Med 2001: 344 : 1058-1066.

Van Harmelen V, Reynisdottir S, Cianflone K, Degerman E, Hoffstedt J, Nilsell K, Sniderman A, Arner P. Mechanisms involved in the regulation of free fatty acid release from isolated human fat cells by acylation-stimulating protein and insulin. J Biol Chem 1999; 274: 18243-18251.

Saleh J, SnidermanAD, Cianflone K. Regulation of plasma fatty acid metabolism. Clin Chim Acta 1999; 286: 163-180.

Saleh J, Blevins JE, Barrett JA, Gietzen DW, Cianflone K. Acylation stimulating protein (ASP) acute effects on postprandial lipemia and food intake in rodents. Int J Ob 2001; 25: 705-713.

Murray I, Sniderman AD, Cianflone K. Enhanced triglyceride clearance with intraperitoneal human acylation stimulating protein in C57BL/6 mice. Am J Physiol Endocrinol Metab 1999; 277: E474-E480.

Xia Z, Sniderman AD, Cianflone K. Acylation-stimulating protein (ASP) deficiency induces obesity resistance and increased energy expenditure in ob/ob mice. J Biol Chem 2002; 277: 45874-45879.

Murray I, Sniderman AD, Cianflone K. Mice lacking acylation stimulating protein (ASP) have delayed postprandial triglyceride clearance. J Lipid Res 1999; 40: 1671-1676.

Halkes CJM, van Dijk H, De Jaegere PPTh, Plokker HWM, Erkelens DW, Castro Cabezas M. Postprandial increase of complement component 3 in normolipidemic patients with coronary artery disease. Effects of expanded dose simvastatin. Arterioscl Thromb Vasc Biol 2001; 21: 1526-1530.

Halkes CJM, Van Dijk H, De Jaegere PPTh, Plokker HWM, Erkelens DW, Castro Cabezas M. Postprandial ketone bodies as marker for hepatic fatty acid flux. Gender differences and associations with abdominal obesity. Arterioscl Thromb Vasc Biol 2003 (in press).

Sniderman AD, Ribalta J, Castro Cabezas M. How should FCHL be defined and how should we think about its causes? Nutr Metabol Cardiovasc Risk Factors 2001; 11: 259-273.

Saleh J, Summers LKM, Cianflone K, Fielding BA, Sniderman AD, Frayn KN. Coordinated release of acylation stimulating protein (ASP) and triacylglycerol clearance by human adipose tissue in vivo in the postprandial period. J Lipid Res 1998; 39: 884-891.

Ylitalo K, Pajukanta P, Meri S, Cantor RM, Mero-Matikainen N, Vakkilainen J, Nuotio I, Taskinen M-R. Serum C3 but not plasma acylation-stimulating protein is elevated in Finnish Patients with familial combined hyperlipidemia. Arterioscl Thromb Vasc Biol 2001; 21: 838-843.

Koistinen HA, Vidal H, Karonen S-K, Dusserre E, Vallier P, Koivisto VA, Ebeling P. Plasma acylation stimulating protein concentration and subcutaneous adipose tissue C3 mRNA expression in nondiabetic and type 2 diabetic men. Arterioscl Thromb Vasc Biol 2001; 21: 1034-1039.

Ozata M, Gungor D, Turan M, Ozisik G, Bingol N, Ozgurtas T, Ozdemir IC. Improved glycemic controls increases fasting plasma acylation-stimulating protein and decreases leptin concentrations in type II diabetic subjects. J Clin Endocrinol Metab 2001; 86: 3659-3664.

Meijssen S, van Dijk H, Verseyden C, Erkelens DW, Cabezas MC. Delayed and exaggerated postprandial complement component 3 response in familial combined hyperlipidemia. Arterioscler Thromb Vasc Biol 2002;22:811-6.

Wetsel RA, Kidsgaard J, Zsigmond E, Liao W, Chan L. Genetic deficiency of acylation stimulating protein (ASP/C3adesArg) does not cause hyperapobetalipoproteinemia in mice. J Biol Chem 1999; 274: 19429-19433.

Charlesworth JA, Peake PW, Campbell LV, Pussell BA, O'Grady S, Tzilopoulos T. The influence of oral lipid loads on acylation stimulating protein (ASP) in healthy volunteers. In J Obes 1998; 22: 1096-1102.

Sniderman AD, Maslowska M, Cianflone K. Of mice and men (and women) and the acylation-stimulating protein pathway. Curr Opinion Lipidol 2000; 11: 291-296.

Madsen HO, Videm V, Svejgaard A, Svennig JL, Garred P. Assciation of mannose-binding lectin deficiency with severe atherosclerosis. Lancet 1998; 352: 959-960.

Oskarsson OO, Vikingsdottir T, Eiriksdottir G, Gudnason V, Valdimarsson H. Is low concentration of mannose binding lectin an independent risk factor for myocardial infarction? Atherosclerosis 2001; suppl 2 (2): 35.

Hegele RA, Busch ChP, Young TK, Connelly PhW, Cao H. Mannose-binding lectin gene variation and cardiovascular disease in Canadian Inuit. Clin Chem 1999; 45: 1283-1285.

Hegele RA, Ban MR, Anderson CM, Spence JD. Infection-susceptibility alleles of mannose-binding lectin are associated with increased carotid plaque area. J Invest Med 2000; 48: 198-202.

Buono C, Come CE, Witztum JL, Maguire GF, Connelly PhW, Carroll M, Lichtman AH. Influence of C3 deficiency on atherosclerosis. Circulation 2002; 105: 3025-3031.

Laine P, Pentikainen MO, Wurzer R, Penttila A, Paavonen T, Meri S, Kovanen PT. Evidence for complement activation in ruptured coronary plaques in acute myocardial infarction. Am J Cardiol 2002; 90: 404-408.

Yasojima K, Schwab C, McGeer EG, McGeer PL. Complement components, but not complement inhibitors, are upregulated in atherosclerotic plaques. Arterioscl Thromb Vasc Biol 2001; 21: 1214-1219.

Nelson RA. The immune-adherence phenomenon. An immunological specific reaction between microorganisms and erythrocytes leading to enhanced phagocytosis. Science 1953; 118: 733-737.

## Claims

1. The use of MBL or a functional fragment and/or derivative and/or functional equivalent thereof in the preparation of a medicament for enhancing the removal of a substance having at least one available hydroxyl group or a complex comprising such a substance from a body fluid.

**2.** Use according to claim 1 wherein said body fluid is blood, lymph or serum.

**3.** Use according to claim 1 or 2, wherein said body fluid is in a subject.

**4.** Use according to any one of claims 1-3, wherein said substance comprises a polyol.

**5.** Use according to any one of claims 1-4, wherein said substance comprises a nucleic acid.

**6.** Use according to claim 5, wherein said complex comprises a nucleosome.

**7.** Use according to any one of claims 1-6, wherein said functional equivalent comprises an antibody or a fragment and/or derivative thereof comprising a complement activating part of said antibody.

**8.** Use according to any one of claims 1-7 wherein said functional equivalent comprises a C3 and/or C4 activator.

**9.** Use according to any one of claims 1-8, for the prevention and/or amelioration of autoimmune diseases.

**10.** Use according to any one of claims 1-8, for the treatment of infectious diseases.

**11.** Use according to claim 9, for the prevention and/or amelioration of SLE.

**12.** A substance comprising a saccharide core, at least one hydrophobic tail and at least one free hydroxyl group, for use in providing a fatty acid complex in a subject with additional free hydroxyl groups.

**13.** A substance comprising a scaffold as presented in fig.1, provided with at least one hydroxyl group and at least one apolar region for use in providing a fatty acid complex in a subject with additional free hydroxyl groups.

**14.** A substance for use according to claim 12, which comprises a peptide.

**15.** A substance for use according to claim 12, 13 or 14, wherein said hydroxyl group and said apolar region are indirectly linked through a bond, which is not hydrolysed in vivo.

**16.** Use according to any one of claims 1-15, wherein the glucose levels in said subject or body fluid are reduced.

**17.** A kit of parts comprising a preparation including MBL or a functional fragment and/or derivative and/or functional equivalent thereof and a preparation comprising a substance with a saccharide core, at least one hydrophobic tail and at least one free hydroxyl group.

**18.** A kit of parts comprising a preparation comprising MBL or a functional fragment and/or derivative and/or functional equivalent thereof and a preparation comprising a substance including a scaffold as presented in fig.1, provided with at least one hydroxyl group and at least one apolar region .

**19.** A substance according to claim 15 comprising a glycopeptide.

**20.** A method to produce an S-glycopeptide, comprising replacing the connecting oxygen atom between the carbohydrate and the peptide backbone by a sulphur atom.

**21.** A combinatorial library comprising S-glycopeptides, obtained by the method of claim 20.

**22.** A substance according to claim 19 wherein said glycopeptide is an S-glycopeptide.

**23.** An S- glycolipid according to claim 22 which is part of the library according to claim 21.

**24.** A method to produce an N-glycopeptide, comprising replacing the connecting oxygen atom between the carbohydrate and the peptide backbone by a nitrogen atom.

**25.** A combinatorial library comprising N-glycopeptides, obtained by the method of claim 24.

**26.** A substance according to claim 19 wherein said glycopeptide is an N-glycopeptide.

**27.** An N- glycolipid according to claim 26 which is part of the library according to claim 25.

**28.** A method to select an S-glycopeptide and/or an N-glycopeptide as an MBL ligand, comprising providing a combinatorial library of S-glycopeptides and/ or N-glycopeptides, further comprising contacting the glycopeptides of said library with a labelled MBL, and selecting the glycopeptide with which the labelled MBL has bonded.

Figure 1. Experimental platforms for sugar attachments

Figure 2. Schematic representation of divalent recognition between carbohydrate-binding protein (CBP) and complex glycan (left side of figure) or simplified synthetic analogue (right side of figure)

Figure 3. Mannose building block with sulphur-containing amino-acids, successfully connected to the solid supports.

Figure 4. Mannosaccharide containing glycopeptides as good mimics of complex ligands, between the MBL-molecule and lipoproteins.

R = certain sequence of fucose, glucosamine, mannose

Figure 5. Examples of scaffolds for incorporation into the membrane of the lipoproteins.

**European Patent Office**

## PARTIAL EUROPEAN SEARCH REPORT

which under Rule 45 of the European Patent Convention shall be considered, for the purposes of subsequent proceedings, as the European search report

**Application Number**

EP 04 07 7013

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 270 199 A (EZEKOWITZ RAYMOND A B) 14 December 1993 (1993-12-14) * column 4, lines 43-54 * * column 8, lines 43-56 * ----- | 1,3,4 | A61K38/16 |
| X | WO 00/69894 A (THIEL STEFFEN ; JENSENIUS JENS CHRISTIAN (DK)) 23 November 2000 (2000-11-23) * page 3, line 21 - page 4, line 32; claims 1,8 * ----- | 1,9,10 | |
| X | WO 03/077937 A (KONGERSLEV LEIF ; NATIMMUNE AS (DK); LARSEN JESPER LUND (DK)) 25 September 2003 (2003-09-25) * page 4, lines 4-11 * * page 30, line 3 - page 31, line 23 * ----- | 1,10 | |
| X | WO 03/090774 A (DEAN MELINDA MARGARET ; MINCHINTON ROBYN MYRA (AU); QUEENSLAND INST ME) 6 November 2003 (2003-11-06) * page 5, lines 1-4 * * page 7, lines 13,14 * * page 18, line 30 - page 20, line 9 * * page 19, line 26 * ----- | 1,8-11 | |

| TECHNICAL FIELDS SEARCHED (Int.Cl.7) |
|---|
| A61K |

-/--

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 4 November 2004 | Engl, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**PARTIAL EUROPEAN SEARCH REPORT**

Application Number

EP 04 07 7013

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | WO 03/033522 A (MATTHIESEN FINN ; NATIMMUNE AS (DK)) 24 April 2003 (2003-04-24) * page 2, lines 25-27 * * page 10, line 16 - page 11, line 21 * * page 28, line 30 - page 29, line 2 * | 1,11 | |
| Y | WEIS, W. I.; DRICKAMER, K.; HENDRICKSON, W.A.: "Structure of a C-type Mannose-Binding Protein Complexed with an Oligosaccharide" NATURE, vol. 360, no. 6400, 12 November 1992 (1992-11-12), pages 127-134, XP002303895 * the whole document * | 1-11,16 | |
| Y | GARRED P ET AL: "MANNOSE-BINDING LECTIN POLYMORPHISMS AND SUSCEPTIBILITY TO INFECTION IN SYSTEMIC LUPUS ERYTHEMATOSUS" ARTHRITIS AND RHEUMATISM, LIPPINCOTT, PHILADELPHIA, US, vol. 42, no. 10, October 1999 (1999-10), pages 2145-2152, XP008018812 ISSN: 0004-3591 * the whole document * | 1-11,16 | **TECHNICAL FIELDS SEARCHED** (Int.Cl.7) |

EPO FORM 1503 03.82 (P04C10)

European Patent
Office

INCOMPLETE SEARCH
SHEET C

Application Number

EP 04 07 7013

Claim(s) searched completely:
--

Claim(s) searched incompletely:
1-11,16

Claim(s) not searched:
-

Reason for the limitation of the search:

The expression "substance having at least one available hydroxyl group or
a complex comprising ..." is not clear since the substance to be removed
is not thereby specified. Furthermore, it is not clear how and under
which circumstances the hydroxy group is required to be available,
therefore, the expression "available" has no defining character.
Therefore, the subject-matter for which protection is sought is not
clear. It can be concluded from the description that not all substances
having hydroxy groups, but only specific DNA fragments are intended to be
removed with MBL. Therefore, the claimed subject-matter also lacks
support in description.

European Patent

Office

---

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-11, 16

European Patent
Office

**LACK OF UNITY OF INVENTION
SHEET B**

Application Number

EP 04 07 7013

The Search Division considers that the present European patent application does not comply with the requirements of unity of invention and relates to several inventions or groups of inventions, namely:

1. claims: 1-11,16

    Use of MBL in th preparation of a medicament for enhancing the removal of a substance having at least one available hydroxy group or a complex comprising such a substance from a body fluid.

    ---

2. claims: 12,14,15,19

    Substance comprising a saccharide core, at least on ehydrophobic tail and at least one free hydroxy group.

    ---

3. claim: 13

    Substance comprising a scaffold as presented in fig. 1, provided with at least one hydroxyl group and at least one apolar region.

    ---

4. claim: 17

    Kit of parts comprising a preparation including MBL and a preparation comprising a substance with a saccharide core, at least one hydrophobic tail and at least one free hydroxyl group.

    ---

5. claim: 18

    Kit of parts comprising a preparation comprising MBL and a preparation comprising a substance including a scaffold as presented in fig. 1, provided with at least one hydroxyl group and at least one apolar region.

    ---

6. claims: 20-23; 28 (part)

    Method to produce an S-glycopeptide.

    ---

7. claims: 24-27; 28 (part)

    Method to produce an N-glycopeptide.

    ---

## ANNEX TO THE EUROPEAN SEARCH REPORT
## ON EUROPEAN PATENT APPLICATION NO.                    EP 04 07 7013

This annex lists the patent family members relating to the patent documents cited in the above-mentioned European search report.
The members are as contained in the European Patent Office EDP file on
The European Patent Office is in no way liable for these particulars which are merely given for the purpose of information.

04-11-2004

| Patent document cited in search report | | Publication date | Patent family member(s) | | Publication date |
|---|---|---|---|---|---|
| US 5270199 | A | 14-12-1993 | AT | 165980 T | 15-05-1998 |
| | | | AU | 2426288 A | 09-03-1989 |
| | | | DE | 3856180 D1 | 18-06-1998 |
| | | | DE | 3856180 T2 | 03-09-1998 |
| | | | DK | 43890 A | 19-02-1990 |
| | | | EP | 0375736 A1 | 04-07-1990 |
| | | | JP | 2504581 T | 27-12-1990 |
| | | | JP | 2868777 B2 | 10-03-1999 |
| | | | NO | 900745 A | 06-04-1990 |
| | | | WO | 8901519 A1 | 23-02-1989 |
| WO 0069894 | A | 23-11-2000 | AT | 264111 T | 15-04-2004 |
| | | | AU | 4393500 A | 05-12-2000 |
| | | | AU | 779404 B2 | 20-01-2005 |
| | | | AU | 4393600 A | 05-12-2000 |
| | | | CA | 2372128 A1 | 23-11-2000 |
| | | | CA | 2372435 A1 | 23-11-2000 |
| | | | CN | 1359420 T | 17-07-2002 |
| | | | CN | 1359298 T | 17-07-2002 |
| | | | DE | 60009896 D1 | 19-05-2004 |
| | | | WO | 0070043 A1 | 23-11-2000 |
| | | | WO | 0069894 A2 | 23-11-2000 |
| | | | DK | 1181038 T3 | 09-08-2004 |
| | | | EP | 1181363 A1 | 27-02-2002 |
| | | | EP | 1181038 A2 | 27-02-2002 |
| | | | EP | 1402897 A2 | 31-03-2004 |
| | | | ES | 2219336 T3 | 01-12-2004 |
| | | | JP | 2002544286 T | 24-12-2002 |
| | | | JP | 2002543837 T | 24-12-2002 |
| | | | NO | 20015481 A | 11-01-2002 |
| | | | NO | 20015506 A | 09-01-2002 |
| | | | NZ | 515717 A | 30-01-2004 |
| | | | NZ | 515718 A | 26-03-2004 |
| | | | PT | 1181038 T | 31-08-2004 |
| | | | US | 2003191052 A1 | 09-10-2003 |
| | | | US | 6562784 B1 | 13-05-2003 |
| | | | US | 6846649 B1 | 25-01-2005 |
| | | | US | 2004229212 A1 | 18-11-2004 |
| WO 03077937 | A | 25-09-2003 | AU | 2003215524 A1 | 29-09-2003 |
| | | | WO | 03077937 A1 | 25-09-2003 |
| | | | EP | 1344533 A1 | 17-09-2003 |
| | | | US | 2004006009 A1 | 08-01-2004 |
| WO 03090774 | A | 06-11-2003 | WO | 03090774 A1 | 06-11-2003 |
| | | | EP | 1501537 A1 | 02-02-2005 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**                EP 04 07 7013

This annex lists the patent family members relating to the patent documents cited in the above-mentioned European search report.
The members are as contained in the European Patent Office EDP file on
The European Patent Office is in no way liable for these particulars which are merely given for the purpose of information.

04-11-2004

| Patent document cited in search report | | Publication date | Patent family member(s) | | Publication date |
|---|---|---|---|---|---|
| WO 03033522 | A | 24-04-2003 | WO | 03033522 A1 | 24-04-2003 |
| | | | EP | 1335932 A1 | 20-08-2003 |
| | | | US | 2004019186 A1 | 29-01-2004 |